(19) Europäisches Patentamt
European Patent Office
Office européen des brevets

(11) **EP 2 728 502 A1**

(12) **EUROPEAN PATENT APPLICATION**

(43) Date of publication:
**07.05.2014 Bulletin 2014/19**

(51) Int Cl.:
**G06F 19/18** (2011.01)  *G06F 19/20* (2011.01)
**G06F 19/24** (2011.01)

(21) Application number: **13169117.2**

(22) Date of filing: **24.05.2013**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**

(30) Priority: **30.10.2012 HU 1200622**

(71) Applicant: **Silicon Computers Kft.
1118 Budapest (HU)**

(72) Inventors:
• **Sárközy, Péter
H-1064 Budapest (HU)**

• **Antal, Péter Dr.
H-1118 Budapest (HU)**
• **Szalai, Csaba
H-1035 Budapest (HU)**
• **Rónai, Zsolt Dr.
H-2030 Érd (HU)**

(74) Representative: **Harangozo, Gabor
Danubia
Patent & Law Office LLC
Bajcsy-Zsilinszky út 16
1051 Budapest (HU)**

(54) **Method and computer program product for genotype classification**

(57) A method for genotype classification comprises the steps of acquiring a pair of scanned images of an SNP sample for a plurality of individuals selected from a population (S100), wherein one image of the image pairs is associated with a first allele and the other image of the image pair is associated with a second allele of the sample; for both images of the associated scanned image pair of each sample, (i) performing pre-processing of the image to remove scanning noises from the image, (ii) obtaining total sample intensity information from the image (S110), (iii) defining a sample boundary to encompass at least a substantial part of the luminous pixels of the image, (iv) matching said sample boundary to the image, and (v) performing a pixel-based processing of the image using the matched sample boundary in order to obtain image quality information with respect to said sample (S120); and based on said sample intensity information and said image quality information of the sample, grouping the samples into discrete clusters of different genotypes (S130).

```
┌─────────────────────────────────────────────┐
│ S100   Acquire image data of samples          │
└─────────────────────────────────────────────┘
                       │
                       ▼
┌─────────────────────────────────────────────┐
│ S110   Obtain sample intensity information     │
└─────────────────────────────────────────────┘
                       │
                       ▼
┌─────────────────────────────────────────────┐
│ S120   Obtain image quality information        │
└─────────────────────────────────────────────┘
                       │
                       ▼
┌─────────────────────────────────────────────┐
│ S130   Group samples into clusters             │
└─────────────────────────────────────────────┘
```

Figure 1

EP 2 728 502 A1

**Description**

The present invention relates to a method for genotype classification, as well as a computer program product performing the method.

Background art

Single nucleotide polymorphisms (SNPs) are the most common type of genetic variation. A SNP is a single base pair mutation at a specific locus in a DNA sequence, usually consisting of two alleles. SNPs are often found to be the biomarkers of many human diseases and are becoming of particular interest in pharmacogenetics.

A SNP is a DNA sequence variation occurring when a single nucleotide - adenine (A), thymine (T), cytosine (C) or guanine (G) - in the genome (or other shared sequence) is different in two individuals of a species (or between paired chromosomes in an individual). For example, the two sequenced DNA fragments from different individuals AAGCCTA and AAGCTTA contain a difference in a single nucleotide on the fifth position of the fragment, meaning that there are two alleles for this SNP, namely C and T. Almost all common SNPs have only two alleles, three or four alleles are relatively very rare.

Within a population, SNPs can be assigned a minor allele frequency (MAF), which is defined as the lowest allele frequency at a locus that has been observed in a particular population. This is simply the lesser of the two allele frequencies for single nucleotide polymorphisms in the common case of two alleles. With respect to the minor allele frequency, there are variations between human populations, so a SNP allele that is common in one geographical or ethnic group may be much rarer in another population or ethnic group. The well-known Hardy-Weinberg principle states that genotype frequencies in a population are constant from generation to generation unless disturbing effects are introduced. In real populations there may be multiple disturbing factors in effect. The Hardy-Weinberg equilibrium is an ideal state that provides a normalized value against which differences can be analyzed. For allele frequencies to be considered static across generations in a population, it must be assumed that there is no mutation, no migration and no emigration, the size of the population is infinite and the genotypes do not produce any selective pressure.

Variations in the DNA sequences of humans (or other species) can affect how humans develop diseases and respond to pathogens, chemicals, drugs, vaccines and other agents. SNPs are also thought to be key enablers in realizing the concept of personalized medicine. However, their greatest importance in biomedical research is for comparing regions of the genome between cohorts (such as with matched cohorts with and without a disease).

SNP's are measured by using oligonucleotides that hybridize specifically to the single-stranded DNA that contains a template specific sequence with a SNP. Primers hybridize to specific amplicons in a multiplex reaction, one base 3' to the SNP sites. The tagged primers are extended in a two-dye system, by incorporation of a fluorescent labeled chain terminating acyclonucleotide. Two-color detection allows determination of the genotype by comparing optical signals reflected from the two fluorescent dyes. Extended primers are then specifically hybridized to unique samples (i.e. DNA fragments) of an individual, wherein the samples are placed on a well in an arrayed arrangement. Well plate manufacturers typically produce wells with 12 or 48 sample places (spots) thereon. The arrayed wells capture the extended hybridizing products and allow simultaneous detection of a plurality SNP allele signals.

The resulting wells containing the hybridized samples at each spot of the wells are illuminated by two narrow-band light sources, typically short-wavelength laser beams, one for each fluorescent dye used. A camera with a CCD sensor is used to produce sample images based on the fluorescence of the samples located on the wells. In order to make the scanning process efficient, all of the wells of a plate are illuminated and scanned simultaneously. Rudimentary noise filtering is then applied to each scanned sample image to remove high-frequency noise, thereby a pair of raw (scanned) images are produced for each sample, wherein one image of the image pair is associated with a first allele and the other image of the image pair is associated with a second allele of the sample. The raw sample images are then processed to gain intensity information on each sample for both alleles thereof. A genotype call is then assigned to each sample based on the relative intensities of the fluorescent dyes.

There are several methods for classification of genotypes in the art. For example, document WO 2004/003234 discloses a solution for classifying the genotypes using the sample intensity values. However, in this documents, as well as in all known genotype classification schemes, grouping of the samples into different genotypes is based on the representation of the samples in a two-dimensional intensity plane corresponding to the two intensity components according to the different fluorescent dyes, and only the positions of the data points in the intensity plane are analyzed to separate the various clusters of different genotype.

It an object of the present invention to further enhance the prior art classification schemes by introducing image quality parameters in the image processing of the raw sample images and to provide a more sophisticated characterization of the samples to provide a more accurate genotype classification than available in the prior art.

Summary of the invention

The above and other objects are achieved by providing a method for genotype classification, the method comprising the steps of:

a) acquiring a pair of scanned images of an SNP sample for a plurality of individuals selected from a population, wherein one image of the image pairs is associated with a first allele and the other image of the image pair is associated with a second allele of the sample,

b) for both images of the associated scanned image pair of each sample,

i) performing pre-processing of the image to remove scanning noises from the image,

ii) obtaining total sample intensity information from the image,

iii) defining a sample boundary to encompass at least a substantial portion of the luminous pixels of the image,

iv) matching said sample boundary to the image,

v) performing a pixel-based image processing of the image using the matched sample boundary in order to obtain image quality information with respect to said sample,

c) based on said sample intensity information and said sample image quality information, grouping the samples into discrete clusters of different genotypes.

Preferred embodiments of the method according to the present invention are defined by the attached dependent claims.

The above and other objects are further achieved by providing a computer program product including computer readable instructions which, when executed by a computer, perform the steps of the method according to the present invention.

Brief description of the drawings

The present invention will now be described through preferred embodiments thereof with reference to the accompanying drawings, in which:

Figure 1 is a flow diagram of the major steps of the method according to the present invention,

Figure 2 is a flow diagram depicting the image processing steps applied in a preferred embodiment of the method according to the present invention,

Figure 3 schematically illustrates a grid template image used in the method according to the present invention,

Figure 4 shows the location of controls spots in a grid template used in a preferred embodiment of the method according to the invention,

Figure 5A illustrates an example of a convolution mask used for spot level template matching in the method of the invention,

Figure 5B illustrates a spot intensity image with the convolution mask shown in Figure 5A,

Figure 6 is a plot diagram illustrating the corrected sample intensity values of SNP samples classified into separate groups of different genotypes,

Figure 7 is a flow diagram of a preferred embodiment of the method according to the present invention, in which allele specific controls and negative controls are additionally used,

Figure 8 shows a sample intensity plot diagram generated by using *a priori* genetic information in the clustering, and

Figure 9 is a flow diagram illustrating the steps of an alternative embodiment of the method according to the present invention.

Detailed description of the preferred embodiments

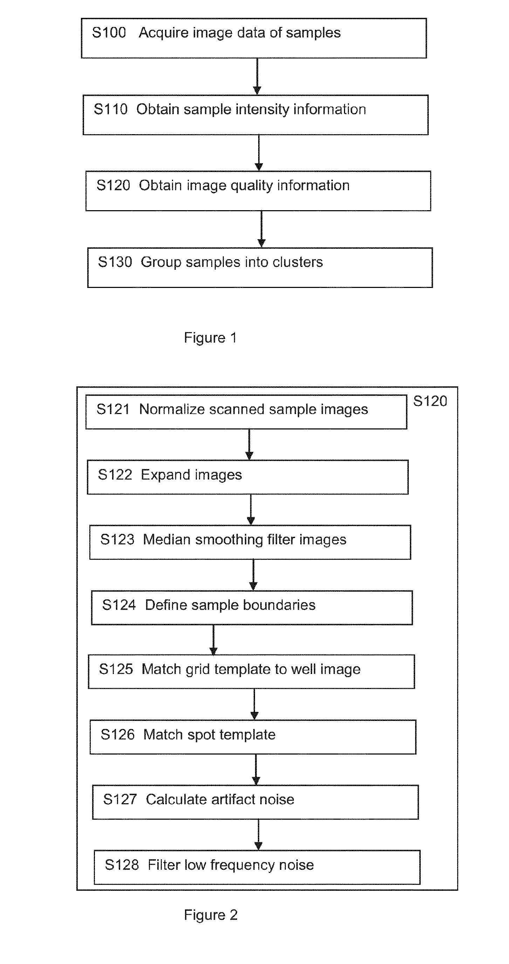

Figure 1 shows a flow diagram depicting the major steps of the method according to the present invention.

In a first step, S100, image data is acquired by scanning the SNP samples by an appropriate scanning device, typically including CCD sensors, thus providing raw sample image data. Throughout the present description, the term "sample" refers to a particular hybridized DNA fragment accommodating at a spot within a well, unless it is otherwise specified.

Next, in step S110, the scanned raw sample images are processed to gain sample intensity information.

When processing the images from the CCD sensor, sometimes calculating the pixel intensities (brightness) of the scanned images may be insufficient to obtain reliable genotyping data from the scanned images, since artifacts and errors, such as scanning noises, dust on the plate, residual chemicals, etc. may distort the scanned image. Therefore in most cases, it is particularly preferred to perform a pre-processing of the scanned sample images to eliminate such noises.

The CCD images of the samples created under the two different narrowband illumination sources are preferably pooled in one well, thereby a plurality of samples belonging to the same illumination source, but associated with various DNA fragments can be processed on one well. As several wells (usually all belonging to different individuals) are grouped in a plate, an efficient scanning and image processing of the samples may be carried out. For each SNP sample, a pair of scanned images is generated, wherein one image of a particular image pair is associated with a first allele and the other image of the image pair is associated with a second allele of the SNP sample.

The scanned sample images provided by the CCD sensor, which are preferably preprocessed to reduce scanning noise, form a basis for the image processing to gain image information on the samples to allow clustering of the samples into genotypes according to the different alleles in a selected DNA fragment.

From the scanned sample images, sample intensity information is gained in a conventional manner in step S110, i.e. the sample images are processed on a pixel basis and the luminosity (or brightness) of the image pixels are typically averaged for each sample to define a sample intensity. The gained sample intensity information of all samples serves as a primary image information for grouping the samples into separate genotype clusters.

It is a novel feature of the method according to present invention that, in step S120, a secondary image information is additionally gained from the scanned sample images in the form of image quality information with respect to the samples to improve the reliability of genotype clustering. The steps of obtaining said image quality information from the scanned sample images will be described below in detail.

On order to produce an improved overall image quality of the scanned sample images for gaining the aforementioned image quality information, it is preferred to perform additional image processing in steps S121 to S128.

First, in step S121, the scanned sample images are normalized. To this end, for each sample image on each well, a raw image of, for example, 16-bit resolution is first read and then these images are normalized to images of 8-bit resolution for a better visibility to the operator. In a further optional step S122, the normalized images are slightly expanded by a few pixels in all of the four image directions (i.e. up, down, left, right) to compensate any misalignment of the scanner head when it was not exactly positioned with respect to the wells.

In the next step S123, median smoothing filtering may be applied to the normalized images to remove a great deal of the high-frequency noise components from the images. The optionally applied median smoothing filtering leaves the characteristic features of the scanned images substantially undisturbed.

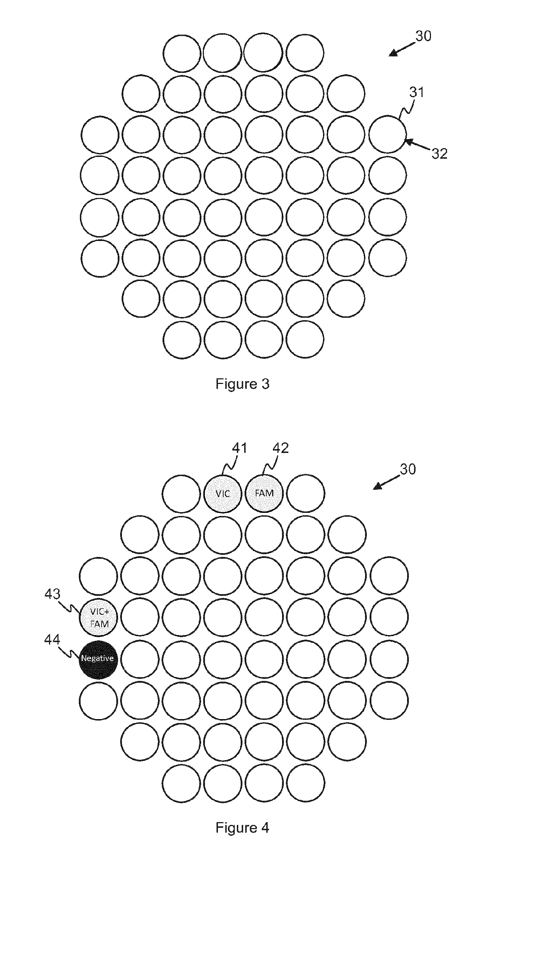

In the next step S124, a sample boundary is defined for each of the sample images so that the sample boundary encompasses at least a substantial portion of the luminous pixels of the sample image, i.e. those pixels having a brightness greater than zero. Since the samples are processed in groups, i.e. there are several samples on a well, the sample boundaries for different samples on the same well are arranged corresponding to the location of the samples, thereby a grid template of multiple sample boundaries is defined for each well. For example, Figure 3 schematically shows a grid template 30 with 48 sample locations (spots) 31, in which a pre-defined sample boundary 32 is arranged at each spot 48. In the grid template, the sample boundaries are arranged and the size of the areas encompassed by the sample boundaries are dimensioned so that at least a substantial portion of the luminous pixels of each sample image belonging to a particular well fall within the sample boundaries of the grid template.

In step S125, the grid template is matched to the image of a sample well (containing arrayed images of several samples) using a predefined template matching algorithm for finding suitable candidate positions for the best alignment of the grid template to the well. Such matching algorithms, like the template matching using a convolution mask (template), are well known in the art.

The grid template is matched on every possible position of the well image. The grid template matching process results in a multi-dimensional parameter space, in which the position of the grid template is to be found using the aforementioned matching algorithm. One possible solution to find the best matching position of the grid template to a well's image is the least square method using the corresponding differential images. In this case, grid template positions with the lowest local minima are selected as candidates for best grid alignment.

Each well may contain four control spots, such as a negative control spot, two homozygous control spots and one heterozygous control spot. The grid template spots corresponding to the aforementioned control spots of the well are depicted in Figure 4, wherein the grid template 30 has a negative control spot 44, two homozygous control spots 41, 42 and one heterozygous control spot 43. For the grid alignment, each well is preferably provided with at least two control spots, in particular the heterozygous control spot and a homozygous control spot.

For each candidate of the best grid position for the well, the control spots and the differential images are evaluated and the best candidate is selected based on the brightest control spots.

The position with the brightest control spot and the least difference from the grid template image for the well will be regarded as the best matching grid template position.

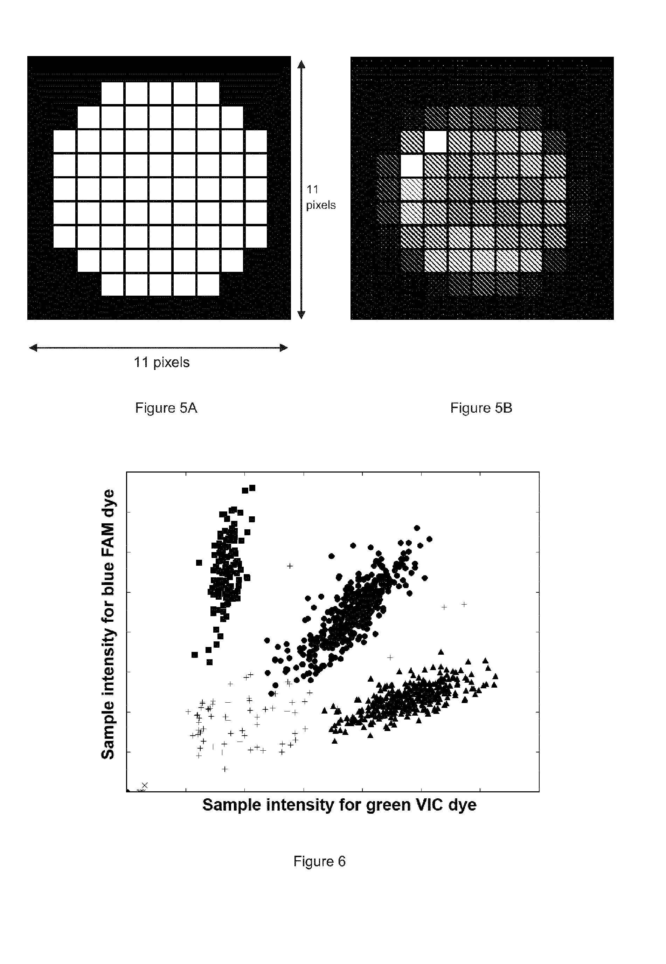

Once the best fitting grid position for the well is determined, another template matching algorithm using a convolution mask is run at the spot level in step S126 for the direct vicinity of the spots in the well to determine the maximum likelihood of the best aligning position of the spot template to the spots of the well. This step is useful because the spots of the well might not be perfectly aligned to the sample boundaries of the grid template due to the low resolution of the sample image. An example of a convolution mask used for spot level template matching is shown in Figure 5A. The illustrated convolution mask contains 11 pixels along each axis, wherein the mask value is 1 for the white pixels and the mask value is 0 for the black pixels. An exemplary spot intensity image using the convolution mask shown in Figure 5A is illustrated in Figure 5B, in which the various patterns of the pixels correspond to different pixel intensity values.

Next, in step S127, an artifact noise is calculated by checking, for example, eight evenly distributed neighbouring pixels around a spot and a noise gradient is generated based on these pixels by linear interpolation between the neighbouring pixels. The estimated artifact noise using this linear model is then subtracted from the sample image at each spot in step S128. This allows the filtering of low frequency noise, such as residual chemicals or wipe marks appearing on the well.

After performing the above image processing steps S121 to S128 to improve image quality, various image quality parameters may be gained from the processed sample images, said parameters together forming a parameter vector used for increasing the reliability of the genotype clustering. The value set of the various image quality parameters is regarded as a secondary image information with respect to the samples of a particular SNP. Some of the most useful image quality parameters are defined below.

**Average intensity:**

The average intensity is the pixel-based average of the pixel brightness values in a sample image for all pixels locating within the matched sample boundary. The average sample intensity, *AvgIntens,* in a spot template of 11 by 11 pixels is defined by the following expression:

$$AvgIntens = \sum_{i=1}^{11} \sum_{j=1}^{11} mask(i,j) * source(i,j) \qquad (1)$$

wherein mask(i,j) is the mask value at the pixel position (i,j) of the spot and source (i,j) is the brightness value of the pixel at position (i,j) of the spot.

**Intensity variance:**

The intensity variance is the pixel-based variance of the pixel brightness values in a sample image for all pixels locating within the matched sample boundary. The sum of each pixel's difference from the average pixel intensity is calculated. The intensity variance, *IntensityVariance,* is defined by the following expression:

$$IntensityVariance = \sum_{i=1}^{11} \sum_{j=1}^{11} mask(i,j) * |source(i,j) - AvgIntens| \qquad (2)$$

wherein *mask(i,j)* is the mask value at the pixel position (i,j) of the spot, *source (i,j)* is the brightness value of the pixel at position (i,j) of the spot, and *AvgIntens* is the average sample intensity of the spot calculated by the expression (1).

**Circularity:**

In order to gain information on the surrounding of a spots, the masking-off of the spot is not performed and circularity of the spot is defined by the expression (4) as the sum of the difference from each spot pixel from the spot average is added to the difference of each surrounding pixel's brightness from the noise floor defined by expression (3):

$$NoiseFloor = \min_{i=1\ldots11, j=1\ldots11}(source(i,j)) \qquad (3)$$

$$Circularity = \sum_{i=1}^{11}\sum_{j=1}^{11} mask(i,j) * (source(i,j) - AvgIntens)^2 + \left(1 - mask(i,j)\right) *$$

$$(source(i,j) - NoiseFloor)^2 \qquad (4)$$

wherein *mask(i,j)* is the mask value at the pixel position (i,j) of the spot, *source (i,j)* is the brightness value of the pixel at position (i,j) of the spot, and *AvgIntens* is the average sample intensity of the spot calculated by the expression (1).

It should be noted that only three image quality parameters have been defined above, but it is obvious for a person skilled in the art that several other image quality parameters may be defined for incorporating in the sample image processing.

Although in the above description, a grid template containing multiple sample boundaries was used in the image processing for practical reasons, the image processing can also be performed using a single sample boundary for one sample based on the same principle.

After obtaining the image quality information for all the sample images, grouping of the samples associated with the same SNP into discrete clusters of different genotypes is carried out in step S130 using said sample intensity information and said sample image quality information relating to the samples.

In a preferred embodiment of the method according to the invention, the quality parameters determined during the image processing are used to calculate a spread value for the Gaussian distribution that the clustering algorithm samples from. The expected value of the Gaussian distribution corresponding to the sample can be the total intensity, or the artifact corrected intensity.

For example in expression (5) each quality parameter is assigned a weight (*w*) and an offset *(a)*, the sum of which is the spread of the Gaussian distribution used by the clustering algorithm.

$$Estimated\ Intensity\ Spread\ \sigma = \sum(w_i * Q_i + a_i) \qquad (5)$$

As a result of the above image processing operations, multiple image quality parameters are also recorded for each sample.

Alternatively, the total sample intensity of a sample may also be determined from all of the pixels within said matched sample boundary in the sample image (also called "non-masked" pixels) after matching the sample boundary to the sample image. The total sample intensity, in this case, is calculated by summing up the brightness value of each non-masked pixels. In this case, the total sample intensity information thus obtained may be used either in addition to or instead of the total sample intensity information determined before matching the sample boundaries (or grid template) to the sample images.

A parameter vector is then associated with each sample, wherein the primary component of the parameter vector is the sample intensity values for each channel (i.e. for the various dyes), and the values of the sample image quality parameters constituting the secondary image information gained from the above described image processing are additional secondary components of said parameter vector.

In the following, the use of the image parameter vector of the samples for grouping the samples into discrete clusters will be described in detail.

In a preferred embodiment of the method according to the invention, a sample confidence level for each sample based on the sample intensity information and the sample image quality information may be defined, and the samples may be grouped into discrete clusters of different genotypes using the sample confidence levels calculated for the samples.

An example of the sample confidence level assigned to sample can be formulated as expression (6). The confidence level is in the range of [0,1].

$$Confidence(i) = \max\left(\quad 1, \frac{(AvgIntens(i) - IntensityVariance(i))}{\sum_j AvgIntens(j))}\right) \qquad (6)$$

In another preferred embodiment of the method according to the invention, the total sample intensity values associated with the samples within the sample image parameter vector are corrected by using at least one of the sample image quality values associated with the samples, and the samples are grouped into discrete clusters of different genotype using the thus obtained corrected sample intensity values. An example of a corrected intensity value can be formulated as expression (7).

$$CorrectedIntensity = \sum_{i=1}^{11}\sum_{j=1}^{11}(AvgIntens - LinearNoiseMap(i,j)) \qquad (7)$$

The corrected intensity values obtained by the above formula are aggregated for each SNP, wherein two sample images belong to the same SNP according to the two dyes.

Figure 6 illustrate the corrected values that are plotted in a coordinate system, in which the X axis corresponds to the VIC (blue) dye, and the Y axis corresponds to the FAM dye (green).

The clusters are preferably separated by using, for example, the well-known K-means clustering algorithm. In this algorithm, each sample is represented by a probabilistic distribution, wherein the probability of its belonging to a particular genotype is specified. Samples that are distant from their respective genotype cluster have lower probabilities.

In Figure 6, each mark corresponds to a sample belonging to a particular genotype, wherein triangles are samples of the homozygous wild genotype, circles are samples of the heterozygous genotype, and squares are samples of the homozygous mutant genotype. Those samples, the probabilities of which do not reach a predefined threshold value, are preferably discarded during clustering. These samples are plotted by cross marks in Figure 6. Those samples that have quality control parameters indicating anomalies in the image processing are also marked by cross marks in Figure 6.

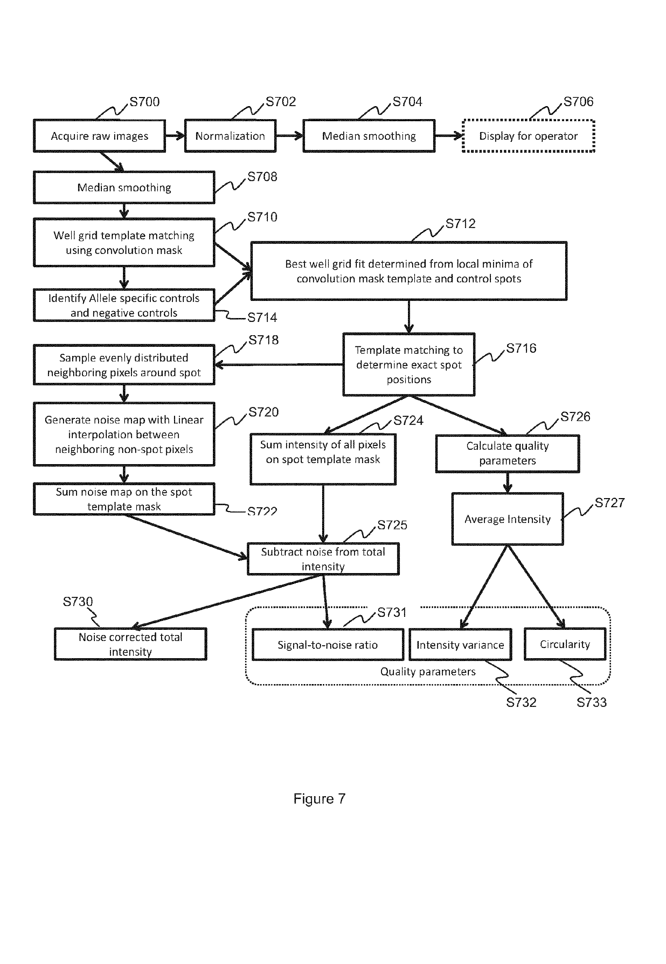

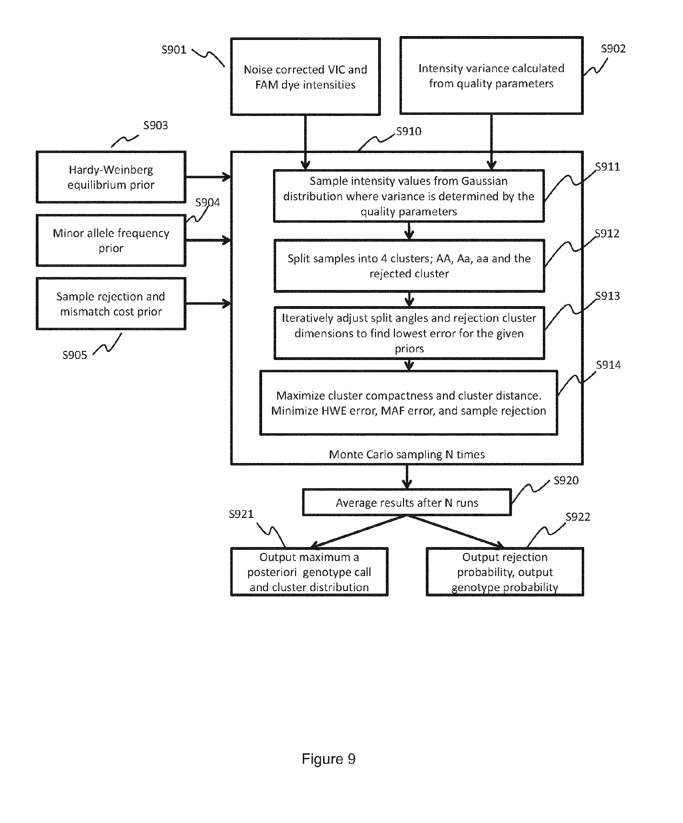

In a particularly preferred embodiment of the method according to the present invention, additional *a priori* information on the particular population is also used in clustering the samples into different genotypes. The *a priori* information may, for example, be an SNP allelic frequency characteristic to the population from which the samples are taken. The flow diagram of such a sample clustering method is illustrated in Figure 7 and Figure 9.

In this embodiment of the method, first the raw sample images are acquired by scanning in step S700. The scanned raw images are normalized in step S702, and then smoothed by median filtering in step S704. The median filtered raw images may optionally be displayed for the operator in step 706.

For the gird template matching, the acquired raw images are first smoothed by median filtering in step S708, followed by the grid template matching using a convolution mask in step S710. During the grid template procedure, the allele specific control values, such as the population specific allele frequency, and negative effect control values are identified in step S714. Using the best fit of the grid template to the well's image, as well as the control spots, the local minima of the convolution mask template (spot template) are identified in step S712.

In step S716 the exact spot positions are determined by matching the grid template to the well's image according to the best fit.

Using the matched grid template, samples are taken from evenly distributed neighbouring pixels around the spots in step S718 to provide non-spot pixels for generating a noise map by linear interpolation between the neighbouring non-spot pixels in step S720. The thus obtained noise maps are summed over the spot template mask (i.e. convolution mask) in step S722.

Using the matched grid template, intensity values of all pixels over the spot template mask are also summed in step S724, and additionally, in step S726 image quality parameters are also calculated to determine an average intensity of the spot image in step S727.

In step S725 the noise is subtracted from the total spot intensity obtained in step S724, and a noise-corrected total spot intensity is thus provided in step S732, using expression (7) for example. As a result of subtraction the spot noise from the total spot intensity, a signal-to-noise ratio of the spot is obtained in step S731.

From the average intensity calculated in step S727, the intensity variance and the circularity are calculated in steps S732 and S733, respectively, using the expressions (2) and (4) above, for example. The thus obtained intensity variance and circularity of the spots, as well as the associated signal-to-noise ratio are regarded as image quality parameters of the spots.

In another preferred embodiment of the method according to the present invention, *a priori* genetic information on the population is further used to allow an optimal separation of the samples into different genotypes. In this embodiment, the step of grouping the samples into discrete clusters of different genotypes may further include the steps illustrated in Figure 9.

In this embodiment, prior constraints about the minor allele frequencies for SNP's characteristic to the population are first provided (see step S904 in Figure 9). Next, an explicit probability for a failed measurement for the DNA of an individual is determined (see step S905 in Figure 9), and then error probabilities for a successfully measured sample are obtained. Finally, a probabilistic estimate about the correspondence of a successfully measured sample to a particular genotype is generated to provide an optimal grouping of the successfully measured samples into discrete clusters of different genotypes.

Assuming that the measurement or at least a subset of our measurements satisfy the Hardy-Weinberg equilibrium principle, this information may also be incorporated to assist in accurate clustering of the genotyping results (see step S903 in Figure 9). In case of a randomly sampled population, the entire sample set may be used, and when performing a case-control study, the control population is used for identifying the Hardy-Weinberg optimal clustering.

A number of samples will have low intensity values (these include the control spots which are designed to give low intensities), for example, due to plate errors or failed amplification or the low quality of the sample DNA. These samples are all ignored at calculating the optimal clustering.

The optimal clustering parameters may be calculated as follows:

1. Samples close to zero intensity (0,0) are discarded, wherein the discard threshold may be adjustable on both axes, and an Euclidean distance metric with separate weights for each channel may be used. The discard area on the cluster plot will result in an elliptical area in general.

2. The *a priori* genetic information is the minor allele frequency for known SNP's, which are available in public databases for various cohorts.

3. For each remaining sample, the intensity ratio for the two channels are calculated, and the samples are classified based on said intensity ratios. The minor allele frequency prior is used to split the samples into three groups based on the well known Hardy-Weinberg equilibrium equation

$$(p^2) + (2pq) + (q^2) = 1$$

4. The split thresholds are displayed on the cluster plot as lines crossing the original sample diagram.

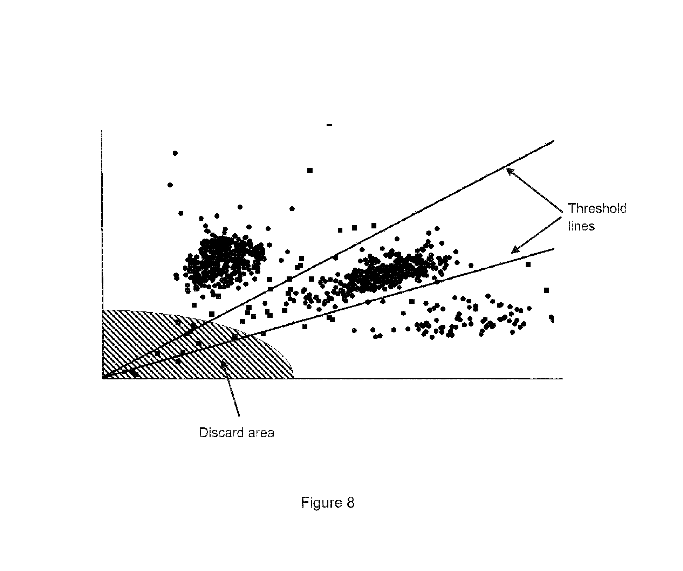

Figure 8 shows a sample intensity plot diagram obtained by the Hardy-Weinberg optimal clustering with a minor allele frequency (MAF) of 0.3.

Figure 9 illustrates the steps of an alternative embodiment of the method according to the invention, wherein a special Monte Carlo sampling of the spot intensities are repeated multiple (N) times with different intensity values sampled from the Gaussian distributions in step S910. During the sampling process, the intensity values are sampled from Gaussian distributions having an expected value obtained as the *CorrectedIntensity* from the noise corrected VIC and FAM dye intensity values in step S901, and an intensity variance calculated from the image quality parameters in step S902. In this context, the terms "sampling" and "sampled" are used in statistical meaning.

In step S912, the samples are split into four clusters, in particular clusters *AA*, *Aa* and *aa*, and a rejection cluster. In the next step S913, the split angles and the dimensions of the rejection cluster are iteratively adjusted to find the lowest error for particular priors, such as the Hardy-Weinberg equilibrium prior, the minor allele frequency prior and the sample rejection and mismatch cost prior, which are provided in steps S903, S904 and S905, respectively.

Next, the cluster compactness and cluster distance are maximized and the values of the aforementioned priors, i.e. the Hardy-Weinberg equilibrium prior, the minor allele frequency prior and the sample rejection and mismatch cost prior, are minimized in step S914.

After the repeated Monte Carlo sampling, the results of each clustering run are averaged in step S920, and the maximum *a posteriori* genotype call and cluster distribution are provided as an output in step S921, and the rejection probability and the probability of each sample corresponding to a specific genotype are provided as an output in step S922.

In another preferred embodiment of the method according to the invention, the method further comprises assigning certainty scores to each classified sample, and a probability of rejection is provided where no genotype is assigned to a sample. According to a second aspect of the present invention, a computer program product is provided, said computer program product including computer readable instructions which, when executed by a computer, perform the steps of the methods according to the present invention.

Example

By using the advantages from the above described advanced image processing techniques for genotype clustering, more accurate genotype calls can be reached as compared to the prior solutions. For example, we were able to assign uncertainty data (or confidence level) to each sample which correlated very closely with the errors resulting from other known methods as well as allowing us to define levels of certainty where we could select samples that passed a minimum certainty threshold.

We compared 768 samples for a single SNP from a 48-well plate, whose results were called by Beckman Coulter's SNPstream genotyping system, with the results called by Applied Biosystem's TaqMan probe based assay and the results of our own image processing application applied to the SNPstream raw image data. The TaqMan probe based system was used as a reference, because its primer is highly optimized for a single SNP and generates very accurate calls, while SNPstream primers are optimized for 48 SNPs at a time, and have a larger margin of error. The SNP chosen for this validation was one that was difficult to assay with SNPstream, because of its low average spot intensities.

SNPstream called 72 SNPs erroneously out of the 768 SNPs compared to the TaqMan assay, while our application called only 56 errors. There were altogether 36 instances where our application had produced calls different from the SNPstream application, and all of these instances had very high associated uncertainty metrics. Most notably the distance from their cluster center and their signal-to-noise ratio showed high uncertainty for these points.

When comparing the calls of SNPstream and the calls of our application, we found that there were only 96 of over 15000 points where the calls differed. All the spots that were called differently had very high uncertainty metrics that were questionable in their classification.

Using the prior information of the MAF of each SNP, the clustering becomes easier, and additional measurement anomalies can also be filtered out.

Although in the above description, specific preferred embodiments of the clustering method according to the present invention have been described in detail with reference to the drawings, it will be understood by those skilled in the art that several other modifications and variants of the method may be carried out without departing the scope of the present invention defined by the appended claims.

**Claims**

1. A method for genotype classification, the method comprising the steps of:

   a) acquiring a pair of scanned images of an SNP sample for a plurality of individuals selected from a population, wherein one image of the image pairs is associated with a first allele and the other image of the image pair is associated with a second allele of the sample (S100),
   b) for both images of the associated scanned image pair of each sample,

      i) performing pre-processing of the image to remove scanning noises from the image,
      ii) obtaining total sample intensity information from the image (S110),
      iii) defining a sample boundary to encompass at least a substantial part of the luminous pixels of the image,
      iv) matching said sample boundary to the image,
      v) performing a pixel-based processing of the image using the matched sample boundary in order to obtain image quality information with respect to said sample (S120),

   c) based on said sample intensity information and said image quality information of the sample, grouping the samples into discrete clusters of different genotypes (S130).

2. The method according to claim 1, wherein before the step iv) of matching, performing a pixel-based normalization and median smoothing filtering of the scanned sample images.

3. The method according to claim 1 or 2, wherein the sample image quality information includes at least one of an average pixel intensity within the matched sample boundary, a variance of the pixel intensity within the matched sample boundary and a circularity of the luminous pixels of the scanned sample image.

4. The method according to any one of claims 1 to 3, wherein when grouping the samples into discrete clusters of different genotypes, *a priori* genetic information on the population is further used to separate the different genotypes and the method further comprises the steps of

- providing prior constraints about minor allele frequencies of the population,
- calculating explicit probability for a failed measurement of a given sample,
- calculating error probabilities for a successfully measured sample, and
- generating a probabilistic estimate about the correspondence of a successfully measured sample to a particular genotype for providing an optimal grouping of the successfully measured samples into discrete clusters of different genotypes.

5. The method according to any one of claims 1 to 4, wherein

- the step of grouping the samples further comprises defining a sample confidence level for each sample based on said sample intensity information and said sample image quality information, and
- the samples are grouped into discrete clusters of different genotypes using said sample confidence levels of the samples.

6. The method according to any one of claims 1 to 5, wherein in addition to step ii), a further total sample intensity is determined for each sample in step v) from all of the pixels falling within said matched sample boundary of the sample.

7. The method according to any one of claims 1 to 6, further comprising the steps of

- assigning certainty scores to each classified sample, and
- providing a probability of rejection where no genotype is assigned to a sample.

8. A computer program product including computer-readable instructions which, when being executed on a computer, perform the steps of the method according to any one of claims 1 to 7.

S100   Acquire image data of samples

S110   Obtain sample intensity information

S120   Obtain image quality information

S130   Group samples into clusters

Figure 1

S120

S121   Normalize scanned sample images

S122   Expand images

S123   Median smoothing filter images

S124   Define sample boundaries

S125   Match grid template to well image

S126   Match spot template

S127   Calculate artifact noise

S128   Filter low frequency noise

Figure 2

Figure 3

Figure 4

11 pixels

11 pixels

Figure 5A                                        Figure 5B

Figure 6

S700
Acquire raw images

S702
Normalization

S704
Median smoothing

S706
Display for operator

S708
Median smoothing

S710
Well grid template matching using convolution mask

S712
Best well grid fit determined from local minima of convolution mask template and control spots

Identify Allele specific controls and negative controls
S714

S718
Sample evenly distributed neighboring pixels around spot

S716
Template matching to determine exact spot positions

S720
Generate noise map with Linear interpolation between neighboring non-spot pixels

S724
Sum intensity of all pixels on spot template mask

S726
Calculate quality parameters

Sum noise map on the spot template mask
S722

S727
Average Intensity

S725
Subtract noise from total intensity

S730
Noise corrected total intensity

S731
Signal-to-noise ratio

Intensity variance

Circularity

Quality parameters
S732      S733

Figure 7

14

Figure 8

S901

Noise corrected VIC and
FAM dye intensities

S902

Intensity variance calculated
from quality parameters

S903

Hardy-Weinberg
equilibrium prior

S904

Minor allele frequency
prior

Sample rejection and
mismatch cost prior

S905

S910

Sample intensity values from Gaussian
distribution where variance is determined by the
quality parameters

S911

Split samples into 4 clusters; AA, Aa, aa and the
rejected cluster

S912

Iteratively adjust split angles and rejection cluster
dimensions to find lowest error for the given
priors

S913

Maximize cluster compactness and cluster distance.
Minimize HWE error, MAF error, and sample rejection

S914

Monte Carlo sampling N times

Average results after N runs

S920

S921

Output maximum a
posteriori genotype call
and cluster distribution

Output rejection
probability, output
genotype probability

S922

Figure 9

Europäisches
Patentamt

European
Patent Office

Office européen
des brevets

# EUROPEAN SEARCH REPORT

Application Number

EP 13 16 9117

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | WO 2004/003234 A2 (APPLERA CORP [US]) 8 January 2004 (2004-01-08) * abstract * * page 7, line 33 - page 8, line 34 * * page 13, line 7 - line 31 * * page 15, line 7 - page 16, line 29 * * claim 1 *  ----- | 1-8 | INV. G06F19/18  ADD. G06F19/20 G06F19/24 |
| A | "PowerPoint on Microarrays: Microarrays IST 444 2009", IST 444 Course Home Page, 3 August 2010 (2010-08-03), XP055099662, Retrieved from the Internet: URL:http://web.archive.org/web/20100803182 703/http://science.marshall.edu/murraye/44 4/444fall2009.html [retrieved on 2014-01-31] * page 38 * * page 46 * * page 49 - page 52 *  ----- | 1-8 | |
| A | B. CARVALHO ET AL: "Exploration, normalization, and genotype calls of high-density oligonucleotide SNP array data", BIOSTATISTICS, vol. 8, no. 2, 22 December 2006 (2006-12-22), pages 485-499, XP055099668, ISSN: 1465-4644, DOI: 10.1093/biostatistics/kxl042 * the whole document *  ----- | 1-8 | TECHNICAL FIELDS SEARCHED (IPC)  G06F |

The present search report has been drawn up for all claims

| Place of search | Date of completion of the search | Examiner |
|---|---|---|
| Munich | 18 February 2014 | Schmitt, Constanze |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

**ANNEX TO THE EUROPEAN SEARCH REPORT
ON EUROPEAN PATENT APPLICATION NO.**

EP 13 16 9117

This annex lists the patent family members relating to the patent documents cited in  the above-mentioned European search report.
The members are as contained in the European Patent Office EDP file on
The European Patent Office is in no way liable for these particulars which are merely given for the purpose of information.

18-02-2014

| Patent document cited in search report | | Publication date | Patent family member(s) | | Publication date |
|---|---|---|---|---|---|
| WO 2004003234 | A2 | 08-01-2004 | AU | 2003247832 A1 | 19-01-2004 |
| | | | CA | 2490766 A1 | 08-01-2004 |
| | | | EP | 1535232 A2 | 01-06-2005 |
| | | | JP | 2005531853 A | 20-10-2005 |
| | | | US | 2004126782 A1 | 01-07-2004 |
| | | | WO | 2004003234 A2 | 08-01-2004 |

EPO FORM P0459

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2004003234 A